# EUROPEAN PATENT SPECIFICATION

(11) **EP 3 804 668 B1**
(45) Date of publication and mention of the grant of the patent: **10.01.2024**
(21) Application number: 20196620.7
(22) Date of filing: 17.09.2020
(51) Int. Cl.: A61F 7/02, A61M 37/00

(54) **BEAUTY INSTRUMENT WITH MASK AND SOFT PHYSIOTHERAPY INSTRUMENT**
SCHÖNHEITSINSTRUMENT MIT MASKE UND WEICHEM PHYSIOTHERAPIEINSTRUMENT
INSTRUMENT DE BEAUTÉ DOTÉ D'UN MASQUE ET INSTRUMENT DE PHYSIOTHÉRAPIE DOUCE

(30) Priority: 11.10.2019 CN 201910965297; 11.10.2019 CN 201910965292; 11.10.2019 CN 201910964750; 11.10.2019 CN 201910965293; 11.10.2019 CN 201910965295; 15.10.2019 CN 201910980369; 15.10.2019 CN 201910980370
(43) Date of publication of application: 14.04.2021
(73) Proprietor: Beijing Funate Innovation Technology Co., Ltd., Beijing (CN)
(72) Inventor: LI, FAN, Beijing (CN); LI, QIAN, Beijing (CN); YU-QUAN, WANG, Beijing (CN)
(74) Representative: Zaboliene, Reda

(56) References cited:
- CN-A- 107 260 390
- CN-U- 207 168 836
- CN-U- 208 838 309
- US-A1- 2012 250 908

## Description

### FIELD

The subject matter herein generally relates to a beauty instrument with mask.

### BACKGROUND

As the living standards being improved, demands for beauty are becoming greater. As such, products of beauty flexible masks and beauty instruments are popular, especially the beauty instruments. Beauty instruments which can produce micro-currents to stimulate human faces are favored by consumers. Existing beauty instruments are hand-held beauty instruments and require users to operate the beauty instruments in front of a mirror. This makes the hand-held beauty instruments inconvenient to use. CN 107 260 390 A discloses a cosmetic facial mask which comprises a first waterproof insulating layer, a second waterproof insulating layer, a carbon nanotube film layer, electrodes, a temperature sensor, a controller, and a resistance adjusting area.

### BRIEF DESCRIPTION OF THE DRAWINGS

Implementations of the present technology will now be described, by way of embodiments, with reference to the attached figures, wherein:
FIG. 1 is a schematic view of a beauty instrument with mask according to a first embodiment.
FIG. 2 is a photo of the beauty instrument with mask according to the first embodiment.
FIG. 3 shows schematic views of functional layers' numberings in the beauty instrument with mask provided by the first embodiment.
FIG. 4 shows a Scanning Electron Microscope (SEM) image of a drawn carbon nanotube film.
FIG. 5 is a schematic view of carbon nanotube segments in the drawn carbon nanotube film.
FIG. 6 shows an SEM image of a flocculated carbon nanotube film.
FIG. 7 shows an SEM image of a pressed carbon nanotube film.
FIG. 8 is a schematic view of a functional layer including a plurality of carbon nanotube wires crossed with each other.
FIG. 9 is a schematic view of a functional layer including a plurality of carbon nanotube wires weaved with each other.
FIG. 10 is a schematic view of a functional layer including a bended and winded carbon nanotube wire.
FIG. 11 is an SEM image of an untwisted carbon nanotube wire.
FIG. 12 is an SEM image of a twisted carbon nanotube wire.
FIG. 13 is a schematic view of a beauty instrument with mask according to a second embodiment.
FIG. 14 is a schematic view of part of a beauty instrument with mask according to a third embodiment.
FIG. 15 is a schematic view of a beauty instrument with mask according to a fourth embodiment.
FIG. 16 is a schematic view of a beauty instrument with mask according to a fifth embodiment.
FIG. 17 is a schematic view of part of a beauty instrument with mask according to the fifth embodiment.
FIG. 18 is a schematic view of a soft physiotherapy instrument according to one embodiment.
FIG. 19 is a schematic view of a clothes using the soft physiotherapy instrument according to one embodiment.
FIG. 20 shows schematic views of functional layers' numberings in the soft physiotherapy instrument provided by the embodiment in FIG. 18.

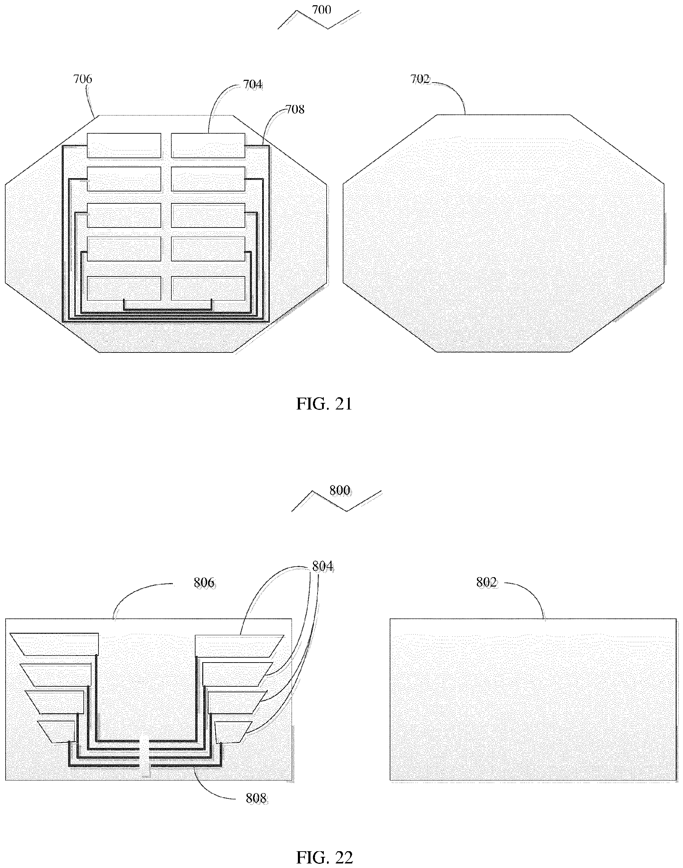

FIG. 21 is a schematic view of a soft physiotherapy instrument according to another embodiment.
FIG. 22 is a schematic view of a soft physiotherapy instrument according to yet another embodiment.

### DETAILED DESCRIPTION

The disclosure is illustrated by way of example and not by way of limitation in the figures of the accompanying drawings in which like references indicate similar elements. It should be noted that references to "another," "an," or "one" embodiment in this disclosure are not necessarily to the same embodiment, and such references mean "at least one."

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

Several definitions that apply throughout this disclosure will now be presented.

The term "contact" is defined as a direct and physical contact. The term "substantially" is defined to be that while essentially conforming to the particular dimension, shape, or other feature that is described, the component is not or need not be exactly conforming to the description. The term "comprising," when utilized, means "including, but not necessarily limited to"; it specifically indicates open-ended inclusion or membership in the so-described combination, group, series, and the like.

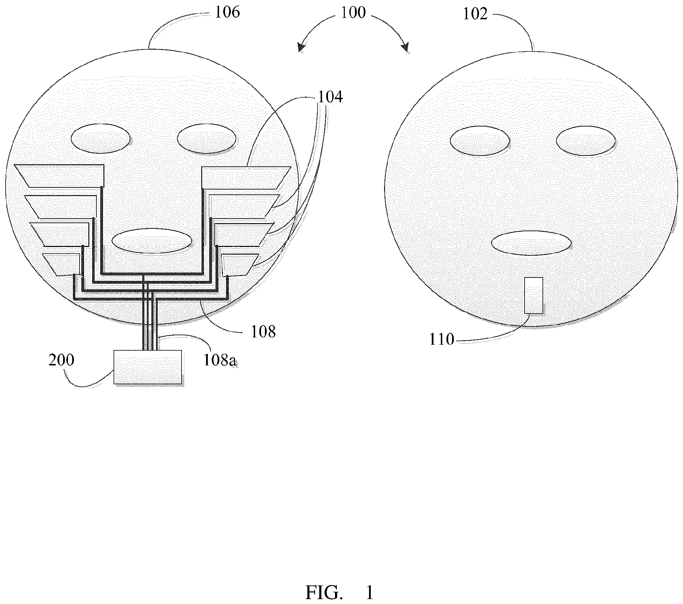

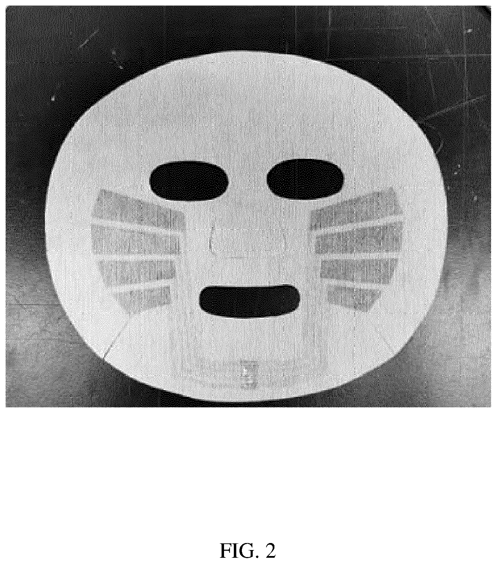

Referring to FIGS. 1 and 2, a beauty instrument with mask 10 according to a first embodiment is provided. The beauty instrument with mask 10 includes a flexible mask 100 and a controller 200 for controlling the flexible mask 100. The flexible mask 100 includes a first flexible layer 102 and a second flexible layer 106 overlapped with each other (for clarity of display, in FIG. 1, the first flexible layer 102 and the second flexible layer 106 are separately shown), the first flexible layer 102 and the second flexible layer 106 have corresponding eye and mouth openings (not labeled). The flexible mask 100 further includes a plurality of functional layers 104 sandwiched between the first flexible layer 102 and the second flexible layer 106, the plurality of functional layers 104 are symmetrically distributed or regularly distributed, and a plurality of electrodes 108, each of the plurality of electrodes 108 is electrically connected with a single functional layer 104 or a pair of functional layers 104. If a quantity of the plurality of electrodes 108 is defined as K (K = 1, 2, 3, 4, 5 ...), then a quantity of the plurality of functional layers 104 is 2K (K = 1, 2, 3, 4, 5 ...) or K. The controller is electrically connected to the K electrodes 108, and the plurality of functional layers 104 in the flexible mask 100 are controlled by the K electrodes 108. In one embodiment, according to FIG. 1, the quantity of the plurality of functional layers 104 is 8 (2K), for example, the quantity of the plurality of electrodes 108 is 4 (K). Each electrode 108 is electrically connected with two functional layers 104. Two ends of the electrode 108 are separately connected with the two functional layers 104. The two functional layers 104 electrically connected with the electrode 108 are symmetrically located on two sides of a user's face.

At least one functional layer 104 can be a plurality of functional layers 104, or one functional layer 104. As shown in FIG. 1, the flexible mask 100 includes 8 functional layers 104. The 8 functional layers 104 are symmetrically distributed at a cheek position of a human face. When the flexible mask 100 includes a plurality of functional layers 104, the position of the functional layer 104 is not limited, and can be a forehead position, a cheek position, an eye below position, a nose position, or the like. The number of the functional layers 104 is not limited and can be adjusted as needed, and may be 2, 8, 15, 20, or the like. An area of each functional layer 104 is not limited and can be adjusted as needed. Adjacent functional layers 104 are spaced apart and insulated from each other.

The controller 200 includes a plurality of function buttons for controlling the flexible mask 100. The controller 200 is electrically connected to the flexible mask 100 through the plurality of electrodes 108. The controller is used to input a voltage between two of the plurality of electrodes 108 to produce current in the plurality of functional layers 104. A circuit is formed between the controller, the two of the plurality of electrodes 108, the plurality of functional layers 104 electrically connected with the two of the plurality of electrodes 108, and the face skin of the user. As such, the current flows through the controller, the two of the plurality of electrodes 108, the plurality of functional layers 104 electrically connected with the two of the plurality of electrodes 108, and the face skin of the user. Each of the plurality of function buttons can control the current magnitude, the frequency of the current, the position of the input current, etc., to control the plurality of functional layers 104 inside the flexible mask 100. The flexible mask 100 can be movably coupled to the controller 200. Optionally, the first flexible layer 102 or the second flexible layer 106 can include a window 110, and the plurality of electrodes 108 a first electrode lead 114 and a second electrode lead 116 are exposed from the window 110 and electrically connected to the controller 200 via a plurality of lead wires 108a. The window 110 is provided with an access port through which the controller 200 is connected to the flexible mask 100. The flexible mask 100 can be replaced as needed. The flexible mask 100 can also be cleaned for reuse.

A material of the first flexible layer 102 or the second flexible layer 106 can be a flexible material such as non-woven fabric, silk, flexible cloth, porous flexible paper, or silica gel, and can be directly attached to a person's face. A thickness of the first flexible layer 102 or the second flexible layer 106 can be set according to actual needs. In this embodiment, the thickness of the first flexible layer 102 or the second flexible layer 106 is in a range from 10 to 100 micrometers. In use of the beauty instrument with mask, the second flexible layer 106 will be directly attached on a face. The second flexible layer 106 has a porous structure.

A material of the electrode 108 can be metal, alloy, indium tin oxide (ITO), antimony tin oxide (ATO), conductive silver paste, conductive polymer, or conductive carbon nanotube. In this embodiment, the K electrodes 108 are all copper wires with a diameter of 1 micrometer. Preferably, an insulating layer can be coated on the surface of each of the K electrodes 108.

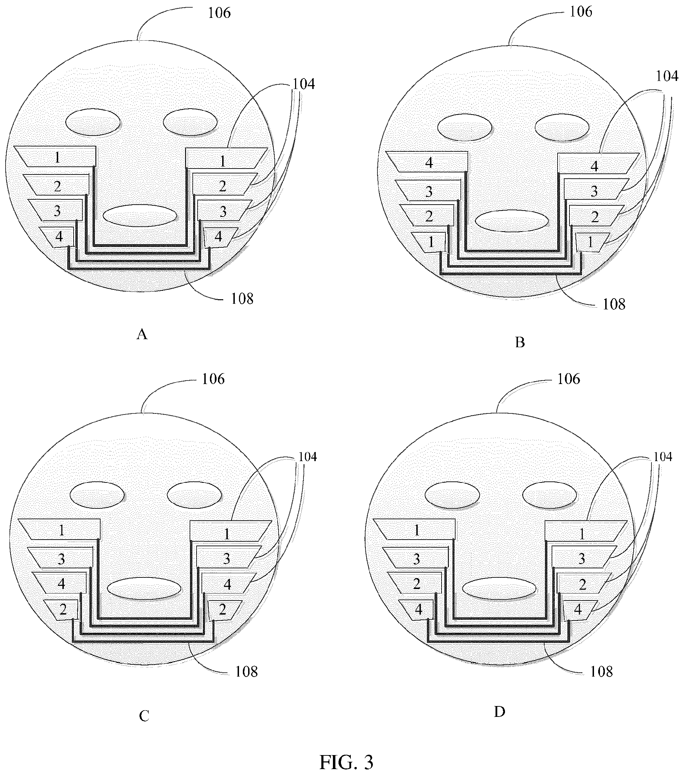

Each electrode 108 corresponds to one functional layer 104 or two functional layers 104. When one electrode 108 corresponds to one functional layer 104, one end of the electrode 108 is electrically connected to the functional layer 104, and the other end is electrically connected to the controller. In this case, the controller can control the one functional layer 104 through the one electrode 108. The numbering of the one electrode 108 corresponds to the numbering of the one functional layer 104. When one electrode 108 corresponds to two functional layers 104, two ends of the electrode 108 are separately electrically connected to one of the two functional layers 104, and a middle part of the electrode 108 is electrically connected to the controller. In this case, the controller can control the two functional layers 104 simultaneously, and the numbering of the one electrode 108 corresponds to the numbering of a pair of functional layers 104. The two functional layers 104 electrically connected with the same electrode 108 have the same numbering. The numbering order of the plurality of electrodes 108 does not represent the position order. That is, the positions of two electrodes 108 with neighbor numbering may be adjacently or at intervals. Since the numbering of the electrode 108 corresponds to the numbering of the functional layer 104, it can be understood that the positions of two pairs of adjacent functional layers 104 or the two adjacent functional layers 104 with neighbor numbering may be adjacently or at intervals. Referring to FIG. 1, in this embodiment, one electrode 108 corresponds to a pair of functional layers 104, and the flexible mask 100 includes four pairs of functional layers 104. Referring to FIGS. 3A to 3D, for example, the four pairs of functional layers 104 are numbered 1, 2, 3, and 4, positions 1, 2, 3, and 4 can be arbitrarily set.

Each of the plurality of functional layers 104 comprises a carbon nanotube layer. In some embodiments, each of the plurality of functional layers 104 can only comprise the carbon nanotube layer. The carbon nanotube layer includes a plurality of carbon nanotubes joined by van der Waals attractive force therebetween. The carbon nanotube layer can be a substantially pure structure of carbon nanotubes, with few impurities. The carbon nanotube layer can be a freestanding structure, that is, the carbon nanotube layer can be supported by itself without a substrate. For example, if at least one point of the carbon nanotube layer is held, the entire carbon nanotube layer can be lifted while remaining its structural integrity.

The carbon nanotubes in the carbon nanotube layer can be orderly or disorderly arranged. The term `disordered carbon nanotube layer' refers to a structure where the carbon nanotubes are arranged along different directions, and the aligning directions of the carbon nanotubes are random. The number of the carbon nanotubes arranged along each different direction can be almost the same (e.g. uniformly disordered). The disordered carbon nanotube layer can be isotropic, namely the carbon nanotube layer has properties identical in all directions of the carbon nanotube layer. The carbon nanotubes in the disordered carbon nanotube layer can be entangled with each other.

The carbon nanotube layer including ordered carbon nanotubes is an ordered carbon nanotube layer. The term 'ordered carbon nanotube layer' refers to a structure where the carbon nanotubes are arranged in a consistently systematic manner, e.g., the carbon nanotubes are arranged approximately along a same direction and/or have two or more sections within each of which the carbon nanotubes are arranged approximately along a same direction (different sections can have different directions). The carbon nanotubes in the carbon nanotube layer can be selected from single-walled, double-walled, and/or multi-walled carbon nanotubes. The carbon nanotube layer may include at least one carbon nanotube film. In other embodiments, the carbon nanotube layer is composed of one carbon nanotube film or at least two carbon nanotube films. In other embodiment, the carbon nanotube layer consists one carbon nanotube film or at least two carbon nanotube films.

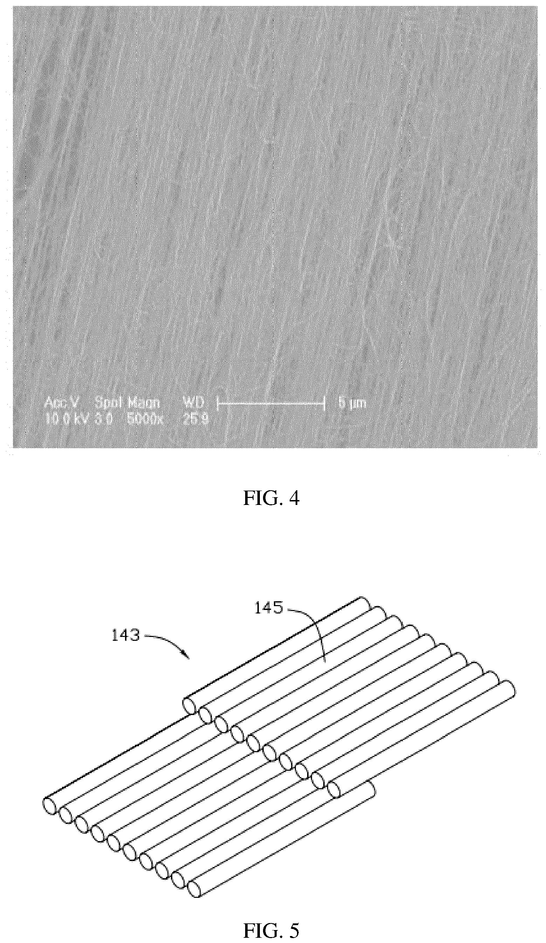

In one embodiment, the carbon nanotube film can be a drawn carbon nanotube film. Referring to FIG. 4, the drawn carbon nanotube film includes a number of successive and oriented carbon nanotubes joined end-to-end by van der Waals attractive force therebetween. The drawn carbon nanotube film is a freestanding film. Each drawn carbon nanotube film includes a number of successively oriented carbon nanotube segments joined end-to-end by van der Waals attractive force therebetween. Referring to FIG. 5, each carbon nanotube segment 143 includes a number of carbon nanotubes 145 substantially parallel to each other, and joined by van der Waals attractive force therebetween. Some variations can occur in the drawn carbon nanotube film. The carbon nanotubes in the drawn carbon nanotube film are oriented along a preferred orientation. The drawn carbon nanotube film can be treated with an organic solvent to increase mechanical strength and toughness of the drawn carbon nanotube film and reduce coefficient of friction of the drawn carbon nanotube film. A thickness of the drawn carbon nanotube film may range from about 0.5 nanometers to about 100 micrometers. The drawn carbon nanotube film can be used as a carbon nanotube layer directly.

The carbon nanotubes in the drawn carbon nanotube film can be single-walled, double-walled, and/or multi-walled carbon nanotubes. The diameters of the single-walled carbon nanotubes may range from about 0.5 nanometers to about 50 nanometers. The diameters of the double-walled carbon nanotubes may range from about 1 nanometer to about 50 nanometers. The diameters of the multi-walled carbon nanotubes may range from about 1.5 nanometers to about 50 nanometers. The lengths of the carbon nanotubes may range from about 200 micrometers to about 900 micrometers.

The carbon nanotube layer may include at least two stacked drawn carbon nanotube films. The carbon nanotubes in the drawn carbon nanotube film are aligned along one preferred orientation, an angle can exist between the orientations of carbon nanotubes in adjacent drawn carbon nanotube films, whether stacked or adjacent. An angle between the aligned directions of the carbon nanotubes in two adjacent drawn carbon nanotube films may range from about 0 degrees to about 90 degrees (e.g. about 15 degrees, 45 degrees, or 60 degrees).

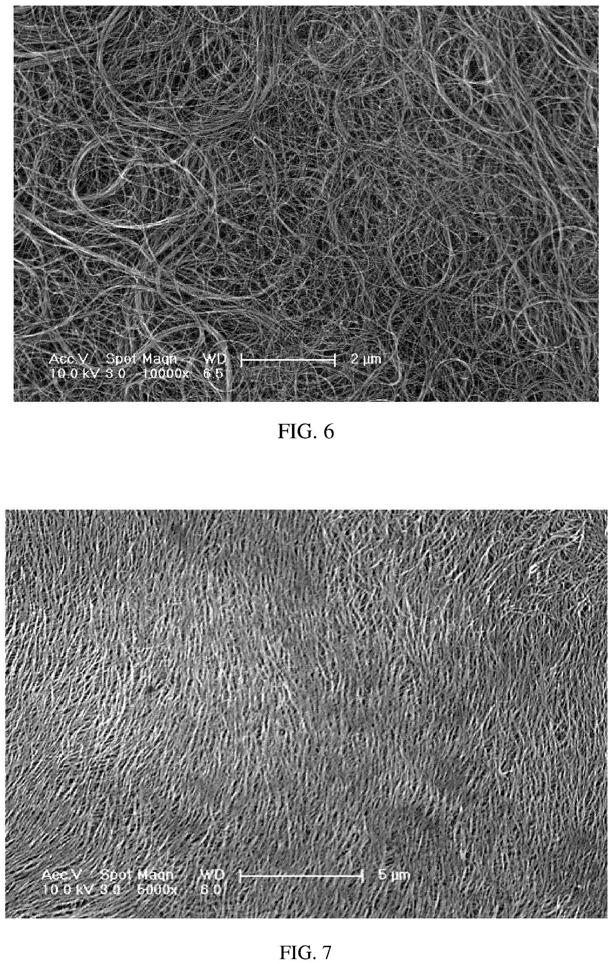

In other embodiments, the carbon nanotube film can be a flocculated carbon nanotube film. Referring to FIG. 6, the flocculated carbon nanotube film may include a plurality of long, curved, and disordered carbon nanotubes entangled with each other. Furthermore, the flocculated carbon nanotube film can be isotropic. The carbon nanotubes can be substantially uniformly dispersed in the flocculated carbon nanotube film. Adjacent carbon nanotubes are acted upon by van der Waals attractive force to obtain an entangled structure with micropores defined therein. Because the carbon nanotubes in the flocculated carbon nanotube film are entangled with each other, the carbon nanotube layer employing the flocculated carbon nanotube film has excellent durability, and can be fashioned into desired shapes with a low risk to the integrity of the carbon nanotube layer. A thickness of the flocculated carbon nanotube film may range from about 0.5 nanometers to about 1 millimeter.

Referring to FIG. 7, in other embodiments, the carbon nanotube film can be a pressed carbon nanotube film. The pressed carbon nanotube film is formed by pressing a carbon nanotube array. The carbon nanotubes in the pressed carbon nanotube film are arranged along a same direction or along different directions. The carbon nanotubes in the pressed carbon nanotube film can rest upon each other. Adjacent carbon nanotubes are attracted to each other and are joined by van der Waals attractive force. An angle between a primary alignment direction of the carbon nanotubes and a surface of the pressed carbon nanotube film is in a range from 0 degrees to 15 degrees. The greater the pressure applied, the smaller the angle obtained. In one embodiment, the carbon nanotubes in the pressed carbon nanotube film are arranged along different directions, the carbon nanotube layer can be isotropic. A thickness of the pressed carbon nanotube film may range from about 0.5 nanometers to about 1 millimeter.

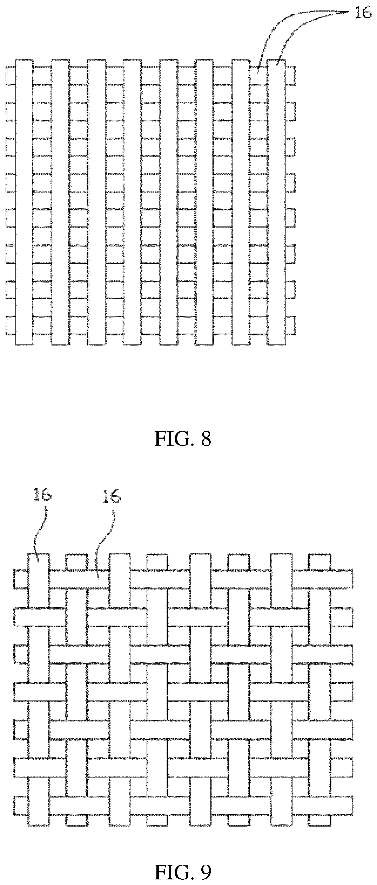

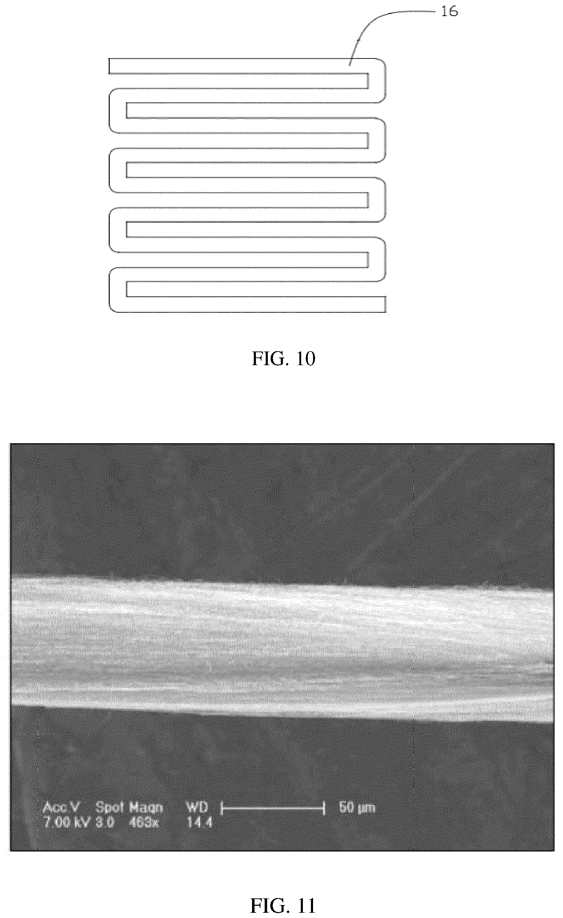

In some embodiments, the carbon nanotube layer may include a plurality of carbon nanotube wires. Referring to FIG. 8, a plurality of carbon nanotube wires 16 can be crossed with each other to form the carbon nanotube layer. Referring to FIG.9, a plurality of carbon nanotube wires 16 can be waved with each other to form the carbon nanotube layer. In other embodiments, the carbon nanotube layer may include only one carbon nanotube wire. Referring to FIG. 10, one carbon nanotube wire 16 can be bended to form the carbon nanotube layer.

The carbon nanotube wire can be untwisted or twisted. Referring to FIG. 11, an untwisted carbon nanotube wire includes a plurality of carbon nanotubes substantially oriented along a same direction (i.e., a direction along the length direction of the untwisted carbon nanotube wire). The untwisted carbon nanotube wire can be a pure structure of carbon nanotubes. The untwisted carbon nanotube wire can be a freestanding structure. The carbon nanotubes are substantially parallel to the axis of the untwisted carbon nanotube wire. In one embodiment, the untwisted carbon nanotube wire may include a plurality of successive carbon nanotube segments joined end to end by van der Waals attractive force therebetween. Each carbon nanotube segment may include a plurality of carbon nanotubes substantially parallel to each other, and combined by van der Waals attractive force therebetween. The carbon nanotube segments can vary in width, thickness, uniformity, and shape. The length of the untwisted carbon nanotube wire can be arbitrarily set as desired. A diameter of the untwisted carbon nanotube wire may range from about 50 nanometers to about 100 micrometers.

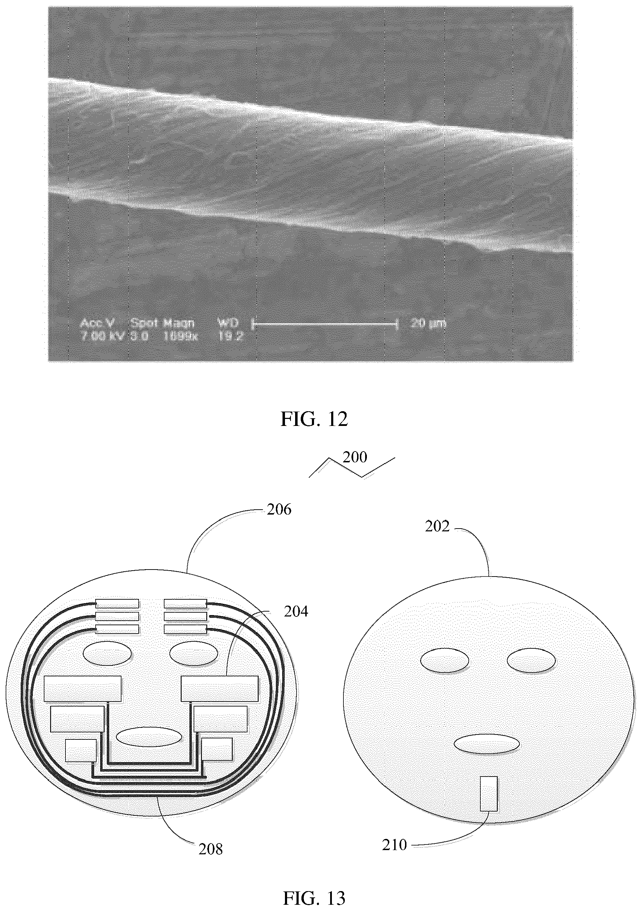

Referring to FIG. 12, a twisted carbon nanotube wire may include a plurality of carbon nanotubes helically oriented around an axial direction of the twisted carbon nanotube wire. The twisted carbon nanotube wire can be a pure structure of carbon nanotubes. The twisted carbon nanotube wire can be a freestanding structure. In one embodiment, the twisted carbon nanotube wire may include a plurality of successive carbon nanotube segments joined end to end by van der Waals attractive force therebetween. Each carbon nanotube segment may include a plurality of carbon nanotubes substantially parallel to each other, and combined by van der Waals attractive force therebetween. The length of the carbon nanotube wire can be set as desired. A diameter of the twisted carbon nanotube wire may range from about 50 nanometers to about 100 micrometers. Furthermore, the twisted carbon nanotube wire can be treated with a volatile organic solvent after being twisted. After being soaked by the organic solvent, the adjacent substantially parallel carbon nanotubes in the twisted carbon nanotube wire will bundle together, due to a surface tension of the organic solvent when the organic solvent volatilizes. The density and strength of the twisted carbon nanotube wire will increase.

The carbon nanotube layer has a better flexibility than the first flexible layer 102 and/or the second flexible layer 106. When the carbon nanotube layer is used as the functional layer 104 in the flexible mask 100, the flexibility of the entire flexible mask 100 is not decreased by the functional layer 104. The carbon nanotube layer has a large strength, as such, no matter how the flexible mask 100 is bent or pulled, and the carbon nanotube layer is not damaged.

In other embodiments, each of the plurality of functional layers 104 can comprise a graphene layer. In some embodiments, each of the plurality of functional layers 104 only comprises the graphene layer. The graphene layer includes at least one graphene. In one embodiment, the graphene layer is a pure structure of graphenes. The graphene layer structure can include a single graphene or a plurality of graphenes. In one embodiment, the graphene layer includes a plurality of graphenes, the plurality of graphenes is stacked with each other and/or located side by side. The plurality of graphenes is combined with each other by van der Waals attractive force. The graphene layer can be a continuous integrated structure. The term "continuous integrated structure" can be defined as a structure that is combined by a plurality of chemical covalent bonds (e.g., sp² bonds, sp¹ bonds, or sp³ bonds) to form an overall structure. A thickness of the graphene layer can be less than 1 millimeter.

In other embodiments, each of the plurality of functional layers 104 can include the carbon nanotube layer and the graphene layer overlapped with each other. The carbon nanotube layer and the graphene layer can be two separated layers.

A beauty instrument with mask according to a second embodiment is provided. The beauty instrument with mask comprises a flexible mask and a controller. Referring to FIG. 13, a flexible mask 200 includes a first flexible layer 202 and a second flexible layer 206, the first flexible layer 202 and the second flexible layer 206 are stacked with each other. The flexible mask 200 further includes a plurality of functional layers 204 sandwiched between the first flexible layer 202 and the second flexible layer 206 and a plurality of electrodes 208 electrically connected with the plurality of functional layers 204. In this embodiment, a quantity of the plurality of functional layers 204 is 12, and a quantity of the plurality of electrodes 208 is 6. Each of the plurality of electrodes 208 is electrically connected with a pair of functional layers 204. As shown in FIG. 13, there are 6 functional layers 204 symmetrically located on a cheek position, and 6 functional layers 204 symmetrically located on a forehead position.

Other characteristics of the beauty instrument with mask in the second embodiment are the same as that of the beauty instrument with mask in the first embodiment.

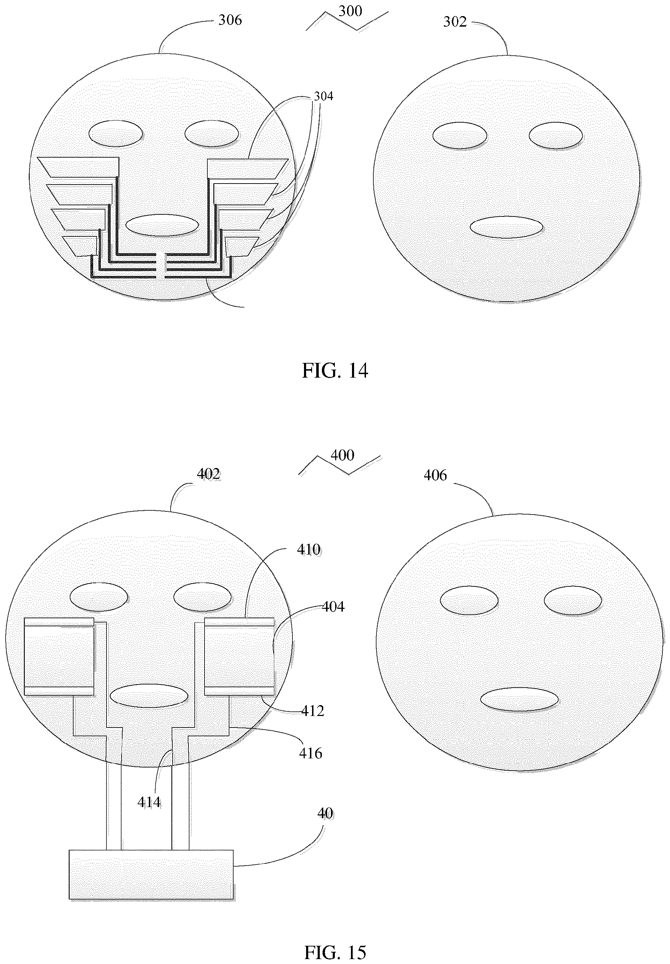

A beauty instrument with mask according to a third embodiment is provided. The beauty instrument with mask comprises a flexible mask and a controller. Referring to FIG. 14, a flexible mask 300 includes a first flexible layer 302 and a second flexible layer 306, the first flexible layer 302 and the second flexible layer 306 are stacked with each other. The flexible mask 300 further includes a plurality of functional layers 304 sandwiched between the first flexible layer 302 and the second flexible layer 306 and a plurality of electrodes 308 electrically connected with the plurality of functional layers 304. In this embodiment, each of the plurality of electrodes 308 is electrically connected with a single functional layer 304. As shown in FIG. 14, there are 8 functional layers 304 symmetrically located on a cheek position, and 8 electrodes 308 are electrically connected with the 8 functional layers 304 in a one by one manner.

Other characteristics of the beauty instrument with mask in the third embodiment are the same as that of the beauty instrument with mask in the first embodiment.

The present disclosure further provides a method of using a beauty instrument with mask, the method comprises the steps of:
Step S1: providing a beauty instrument with mask, the beauty instrument with mask comprises a flexible mask and a controller;
Step S2: applying the flexible mask on a user's face; and
Step S3: turning on the controller and selecting a function button on the controller, inputting a current to a plurality of functional layer in the flexible mask, and stimulating face skin with the current.

In step S1, the beauty instrument with mask is any one of the beauty instrument with masks discussed above.

Alternatively, before step S2, the flexible mask can be further infiltrated with a liquid, that is, before the flexible mask of the beauty instrument with mask is applied on the user's face. The liquid can be a cosmetic liquid.

In step S3, the controller includes a plurality of function buttons for controlling the flexible mask. The controller can control the functional layer inside the flexible mask to simultaneously stimulate the face skin, or selectively control a certain functional layer or some certain functional layers to simultaneously stimulate the face skin. For example, when the functional layers are located at a forehead position, a cheek position, and a chin position, the controller can control the functional layers in the above positions to circulate stimulate the face skin in the order of the forehead position, the cheek position, and the chin position.

In use of the beauty instrument with mask, a voltage is applied to two pairs of functional layers or two functional layers via two electrodes, and a micro-current will be input through the two electrode to the two pairs of functional layers or the two functional layers, and face skin between or under the two pairs of functional layers or the two functional layers will be stimulated by the micro-current. The voltage applied on each two electrodes can be kept for a power-on time, and the voltage is stop for a dwell time, then the voltage is applied to another two electrodes for another power-on time. The voltage can be applied to two electrodes in an order 1 and 2, 2 and 3, 3 and 4 ... K-1 and K (K is the numbering of each electrode), so that the two pairs of functional layers corresponding to each two electrodes are cyclically input current, and the face skin corresponding the two pairs of functional layers are cyclically stimulated. The numbers of the two pairs of functional layers are adjacent, such as numbers 2 and 3, which does not mean that the positions of the two pairs of functional layers are adjacent. The positions of the two pairs of functional layers adjacent to each other can be arbitrarily set according to actual needs. Referring to FIG. 3A to FIG. 3D, in these embodiments, each electrode 108 corresponds to a pair of functional layers 104, and the flexible mask 100 includes four pairs of functional layers 104. The four electrodes 108 are numbered 1, 2, 3, and 4, and the four pairs of functional layers 104 are numbered 1, 2, 3, and 4. Positions 1, 2, 3, and 4 can be arbitrarily set, for example, 3A to 3D in FIG. 3. In the application, the electrodes 108 are energized according to the circulation pattern of the electrodes numbered 1 and 2, 2 and 3, and 3 and 4, thereby sequentially or selectively generating micro-currents in the two pairs of functional layers, which in turn stimulate the face skin.

In one embodiment according to FIG. 3B, in use of the beauty instrument with mask, the electrodes 108 are energized according to the circulation pattern of the electrodes numbered 1 and 2, 2 and 3, and 3 and 4, thereby sequentially or selectively generating micro-currents in the two pairs of functional layers, which in turn stimulate the face skin. In this embodiment, the power-on time of each pair of electrodes 108 is 1 s and the dwell time is 1 s. That is, with a cycle of 2 s, the power is first applied for 1 s, and then stopped for 1 s, and this cycle is performed. Among them, the voltage applied on each two electrodes is in a range of 20V-36V and the frequency of the voltage is 90Khz.

The flexible mask can be movably coupled to the controller. The flexible mask defines an access at the window position on the first flexible layer or the second flexible layer, and the controller is connected to the flexible mask through the access. The flexible mask can be changed as needed. The flexible mask can also be cleaned to achieve re-use purpose.

Compared with the prior art, the beauty instrument with mask provided by the present invention has the following advantages: first, it can directly fit on a user's face without the need to hold it by hand, which frees the user's hands. Secondly, through controlling a circuit by the controller, the skin on the user's face can be selectively stimulated, and the face parts to be stimulated can be selected more accurately without causing facial asymmetry. Third, the carbon nanotube layer is used as the functional layer, the carbon nanotube layer has a better flexibility than the first flexible layer or / and the second flexible layer, and the flexibility of the entire flexible mask will not be reduced due to the setting of the functional layers, the flexible mask can fit on the user's face well, and the user has a high comfort degree. Fourth, the carbon nanotube layer is used as a functional layer, a strength of the carbon nanotube layer is relatively large, no matter how to bend and pull or clean the flexible mask, the carbon nanotube layer will not be damaged, and the flexible mask has a long life.

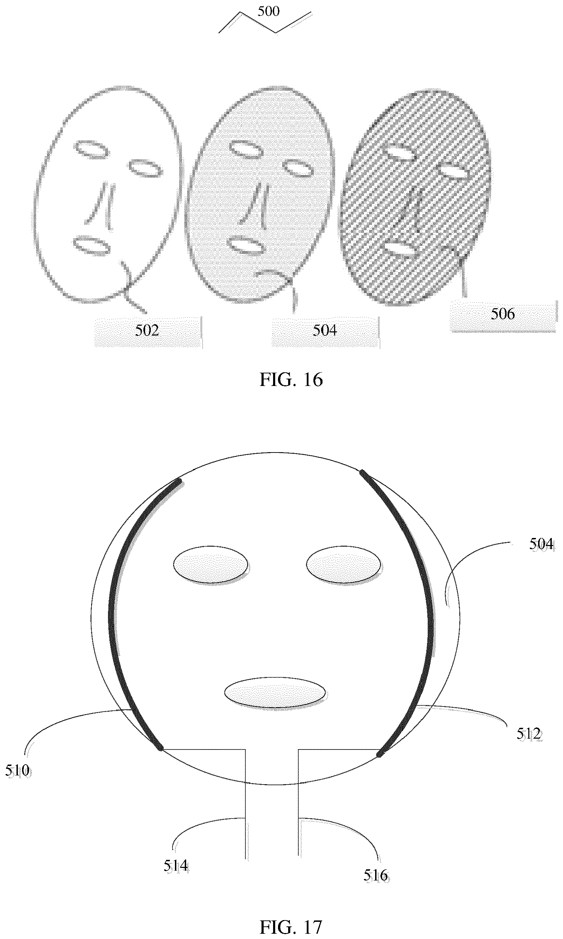

Referring to FIG. 15, a beauty instrument with mask according to a fourth embodiment is provided. The beauty instrument with mask includes a flexible mask 400 and a controller 40 for controlling the flexible mask 400. The flexible mask 400 includes a first flexible layer 402 and a second flexible layer 406 overlapped with each other (for clarity of display, in FIG. 16, the first flexible layer 402 and the second flexible layer 406 are separately shown), the first flexible layer 402 and the second flexible layer 406 have corresponding eye and mouth openings (not labeled); and at least a heating layer 404 located between the first flexible layer 402 and the second flexible layer 406; at least one first electrode 410 and at least one second electrode 412, each heating layer 404 is electrically connected with one first electrode 410 and one second electrode 412; at least one first electrode lead 414 and at least one second electrode lead 414, one first electrode 410 is electrically connected to one first electrode lead 414, and one second electrode 412 is electrically connected to one second electrode lead 416.

The at least one heating layer 404 can be a plurality of heating layers 404, or one heating layer 404. As can be shown in FIG. 15, the flexible mask 400 includes two heating layers 404. The two heating layers 404 are symmetrically distributed at a cheek position of a human face. When the flexible mask 400 includes a plurality of heating layers 404, the position of the heating layer 404 is not limited, and can be a forehead position, a cheek position, an eye below position, a nose position, or the like. The number of the heating layers 404 is not limited and can be adjusted as needed, and may be 2, 8, 15, 20 or the like. Adjacent heating layers 404 are spaced apart and insulated from each other.

The controller 40 includes a plurality of function buttons for controlling the flexible mask 400. The controller 40 is electrically connected to the flexible mask 400 through the at least one first electrode lead 414 and the at least one second electrode lead 416. Each function button can control the current magnitude, the frequency of the current, the position of the input current, etc., to control the heating layer 404 inside the flexible mask 400 to achieve the purpose of heating. The flexible mask 400 can be movably coupled to the controller 40. Optionally, the first flexible layer 402 or the second flexible layer 406 can include a window, and the first electrode lead 414 and the second electrode lead 416 are exposed from the window and electrically connected to the controller. Please referring to FIG. 17, in one embodiment, the second flexible layer 406 defines a window 420, In this embodiment, the flexible mask 400 includes a window 408 defined by the first flexible layer 402. The window 408 is provided with an access port through which the controller is connected to the flexible mask 400. The flexible mask 400 can also be cleaned for reuse.

A material of the first flexible layer 402 or the second flexible layer 406 can be non-woven fabric, silk, flexible cloth, porous flexible paper, or silica gel, and can be directly attached to a person's face. The first flexible layer 402 or the second flexible layer 406 can be a porous structure or a non-porous structure.

The heating layer 404 comprises the carbon nanotube layer or is the carbon nanotube layer. Characteristics of the carbon nanotube layer have been discussed above. In other embodiments, the heating layer 404 comprises the graphene layer or is the graphene layer. Characteristics of the graphene layer have been discussed above. In yet another embodiment, the hearting layer 404 comprises the carbon nanotube layer and the graphene layer overlapped with each other.

Reference to FIG. 16 again, each heating layer 404 is electrically coupled with a first electrode 410 and a second electrode 412. The first electrode 410 and the second electrode 412 are separately located at both ends of the heating layer 404 and are located on a surface of the heating layer 404. The first electrode 410 and the second electrode 412 are directly located the surface of the heating layer 404. In use, a voltage is applied between the first electrode 410 and the second electrode 412, and a current flows inside the heating layer 404 to generate heat. The voltage between the first electrode 410 and the second electrode 412 can be controlled by the controller 10, and a temperature of the heating layer 404 is controlled. By adjusting the controller 40, it is also possible to selectively control which heating layer 104 is heated to selectively heat the face region.

A beauty instrument with mask according to a fifth embodiment is provided. The beauty instrument with mask comprises a flexible mask and a controller. Referring to FIG. 16 and FIG. 17, the flexible mask 500 includes a first flexible layer 502, a second flexible layer 506, the first flexible layer 502 and the second flexible layer 506 are stacked with each other; at least one heating layer 504 located between the first flexible layer 502 and the second flexible layer 506. In this embodiment, only one heating layer 504 located between the first flexible layer 502 and the second flexible layer 506. The heating layer 504 has corresponding openings for the eyes and mouth. The first electrode 510 and the second electrode 512 are respectively located at two ends of the heating layer 504, and are arc-shaped conductive films that match the heating layer 504. The first electrode 510 and the second electrode 512 are electrically connected to the controller (not shown) through a first electrode lead (not shown) and a second electrode lead (not shown). The beauty instrument with mask provided in this embodiment can realize full face heating when working.

Other characteristics of the beauty instrument with mask in the fifth embodiment are the same as that of the beauty instrument with mask in the fourth embodiment.

The present invention further provides a method of using the beauty instrument with mask, the method comprises the steps of:
Step S1: providing the beauty instrument with mask;
Step S2: applying the flexible mask of the beauty instrument with mask on a user's face; and
Step S3: turning on the controller and selecting a function button on the controller, inputting a current to the at least one heating layer in the flexible mask, and heating the at least one heating layer.

In the step S1, the beauty instrument with mask is the beauty instrument with masks discussed the fourth embodiment or the fifth embodiment.

Alternatively, before step S2, the flexible mask can be further infiltrated with a liquid, that is, before the flexible mask of the beauty instrument with mask is applied on the user's face.

In step S3, the controller includes a plurality of function buttons for controlling the flexible mask. The controller can control the heating layer inside the flexible mask to simultaneously heat, or selectively control a certain heating layer or some certain heating layers. For example, when the heating layers are located at a forehead position, a cheek position and a chin position, the controller can control the heating layers in the above positions to circulate heat in the order of the forehead position, the cheek position, and the chin position.

Compared with the prior art, the beauty instrument with mask provided in the fourth embodiment or the fifth embodiment has the following advantages: first, it can directly fit on a user's face without the need to hold it by hand, which frees the user's hands. Secondly, through controlling a circuit by the controller, the skin on the user's face can be selectively stimulated, and the face parts to be stimulated can be selected more accurately without causing facial asymmetry. Third, the carbon nanotube layer is used as the heating layer, the carbon nanotube layer has a better flexibility than the first flexible layer or / and the second flexible layer, and the flexibility of the entire flexible mask will not be reduced due to the setting of the heating layers, the flexible mask can fit on the user's face well, and the user has a high comfort degree. Fourth, the carbon nanotube layer is used as a heating layer, a strength of the carbon nanotube layer is relatively large, no matter how to bend and pull or clean the flexible mask, the carbon nanotube layer will not be damaged, and the flexible mask has a long life.

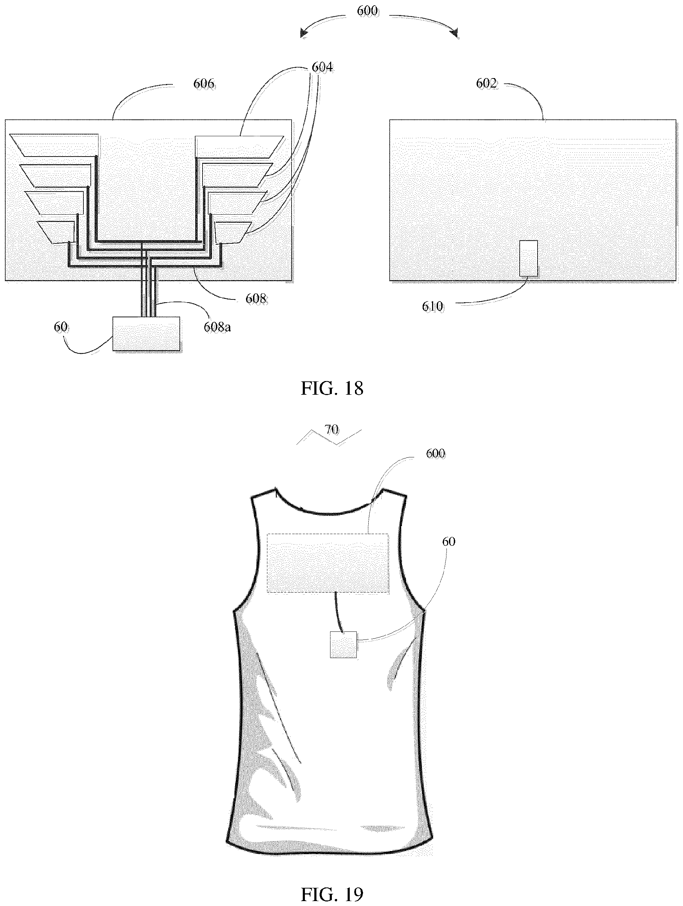

Referring to FIG. 18, a soft physiotherapy instrument (not labeled) according to one embodiment is provided. The soft physiotherapy instrument includes a flexible sheet 600 and a controller 60 for controlling the flexible sheet 600. The flexible sheet 600 includes a first flexible layer 602 and a second flexible layer 606 overlapped with each other (for clarity of display, in FIG. 19, the first flexible layer 602 and the second flexible layer 606 are separately shown), a plurality of functional layers 604 sandwiched between the first flexible layer 602 and the second flexible layer 606, the plurality of functional layers 604 are symmetrically distributed or regularly distributed, and a plurality of electrodes 608, each of the plurality of electrodes 608 is electrically connected with a single functional layer 604 or a pair of functional layers 604. If a quantity of the plurality of electrodes 108 is defined as K (K = 1, 2, 3, 4, 5 ...), then a quantity of the plurality of functional layers 104 is 2K (K = 1, 2, 3, 4, 5 ...) or K. The controller is electrically connected to the K electrodes 108, and the plurality of functional layers 604 in the flexible sheet 600 are controlled by the K electrodes 608. In one embodiment, according to FIG. 19, the quantity of the plurality of functional layers 604 is 8 (2K), for example, the quantity of the plurality of electrodes 608 is 4 (K). Each electrode 608 is electrically connected with two functional layers 604. Two ends of the electrode 608 are separately connected with the two functional layers 604. The two functional layers 604 electrically connected with the electrode 608 are symmetrically located. Each of the plurality of functional layers 604 comprise the carbon nanotube layer. Characteristics of the carbon nanotube layer have been discussed above.

The controller 60 includes a plurality of function buttons for controlling the flexible sheet 600. The controller 60 is electrically connected to the flexible sheet 600 through the plurality of electrodes 608. The controller 600 is used to input a voltage between two of the plurality of electrodes 608 to produce a current in the plurality of functional layers 604. A circuit is formed between the controller 60, the two of the plurality of electrodes 608, the plurality of functional layers 604 electrically connected with the two of the plurality of electrodes 608, and skin of a user. As such, the current flows through the controller 60, the two of the plurality of electrodes 608, the plurality of functional layers 604 electrically connected with the two of the plurality of electrodes 608, and the skin of the user. Each of the plurality of function buttons can control a current magnitude, a frequency of the current, a position of the input current, etc., to control the plurality of functional layers 604 inside the flexible sheet 600. The flexible sheet 600 can be movably coupled to the controller 60. Optionally, the first flexible layer 602 or the second flexible layer 606 can include a window 610, and the plurality of electrodes 608 are exposed from the window 610 and electrically connected to the controller 60 via a plurality of electrode lead wires 608a. The window 610 is provided with an access port through which the controller 60 is connected to the flexible sheet 600. The flexible sheet 600 can be replaced as needed. The flexible sheet 600 can also be cleaned for reuse.

The flexible sheet 600 can be applied to any part of the human body, such as the back, legs, knees, and abdomen. The flexible sheet 600 can also be a part of clothes or other wearables. Please refer to FIG. 19, the flexible sheet 600 can be arranged on a clothes 70 to serve as a shoulder and neck physiotherapy device, and the controller 60 can be built into clothing. It can be understood that, in other embodiments, the controller may also be placed outside the clothes. The flexible sheet 600 can also be directly made into clothing or other wearing objects.

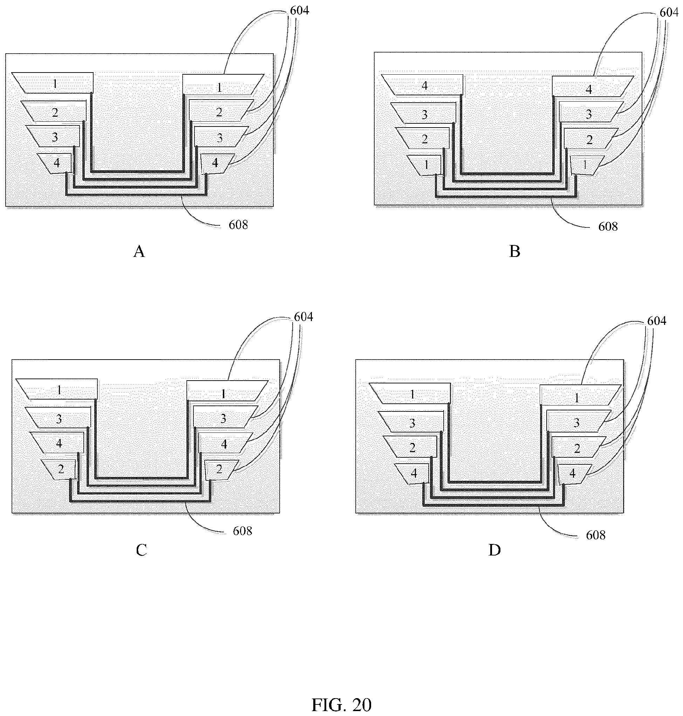

In use of the soft physiotherapy instrument, a voltage is applied to two pairs of functional layers or two functional layers via two electrodes, and a micro-current will be input through the two electrode to the two pairs of functional layers or the two functional layers, and skin between or under the two pairs of functional layers or the two functional layers will be stimulated by the micro-current. The voltage applied on each two electrodes can be kept for a power-on time, and the voltage is stop for a dwell time, then the voltage is applied to another two electrodes for another power-on time and another dwell time. The voltage can be applied to two electrodes in an order 1 and 2, 2 and 3, 3 and 4 ... K-1 and K (K is the numbering of each electrode), so that the two pairs of functional layers corresponding to each two electrodes are cyclically input current, and the skin corresponding the two pairs of functional layers are cyclically stimulated. The numbers of the two pairs of functional layers are adjacent, such as numbers 2 and 3, which does not mean that the positions of the two pairs of functional layers are adjacent. The positions of the two pairs of functional layers adjacent to each other can be arbitrarily set according to actual needs. Referring to FIG. 20A to FIG. 20D, in these embodiments, each electrode 608 corresponds to a pair of functional layers 604, and the flexible sheet 600 includes four pairs of functional layers 604. The four electrodes 608 are numbered 1, 2, 3, and 4, and the four pairs of functional layers 604 are numbered 1, 2, 3, and 4. Positions 1, 2, 3, and 4 can be arbitrarily set, for example, 20A to 20D in FIG. 20. In the application, the electrodes 608 are energized according to the circulation pattern of the electrodes numbered 1 and 2, 2 and 3, and 3 and 4, thereby sequentially or selectively generating micro-currents in the two pairs of functional layers, which in turn stimulate the skin.

In one embodiment according to FIG. 20B, in use of the soft physiotherapy instrument, the electrodes 608 are energized according to the circulation pattern of the electrodes numbered 1 and 2, 2 and 3, and 3 and 4, thereby sequentially or selectively generating micro-currents in the two pairs of functional layers, which in turn stimulate the skin. A working time of each pair of electrodes 608 is defined, during the working time, an alternating current (AC) is applied on the each pair of electrodes 608.

The flexible sheet can be movably coupled to the controller. The flexible sheet defines an access at the window position on the first flexible layer or the second flexible layer, and the controller is connected to the flexible sheet through the access. The flexible sheet can be changed as needed. The flexible sheet can also be cleaned to achieve re-use purpose.

Compared with the prior art, the soft physiotherapy instrument provided by the present invention has the following advantages: First, the flexible sheet has a strong flexibility due to the use of carbon nanotube materials, which can be directly attached to human skin, and the human body has a strong sense of comfort. Second, because the carbon nanotube material can be made into any area as required, the area of the flexible sheet can be set freely, the area acting on the human body can be set as needed, or it can be directly made into a physical therapy clothing. The soft physiotherapy instrument has a wider range of applications. Third, the carbon nanotube layer is used as a functional layer, a strength of the carbon nanotube layer is large, the flexible sheet will not be damaged due to the carbon nanotube layer no matter of how it is bent and pulled, or cleaned.

The exemplary embodiments shown and described above are only examples. Many such details are neither shown nor described. Even though numerous characteristics and advantages of the present technology have been set forth in the foregoing description, together with details of the structure and function of the present disclosure, the disclosure is illustrative only, and changes may be made in the detail, including in matters of shape, size, and arrangement of the parts within the principles of the present disclosure, up to and including the full extent established by the broad general meaning of the terms used in the claims. It will therefore be appreciated that the exemplary embodiments described above may be modified within the scope of the claims.

## Claims

1. A soft physiotherapy instrument, comprising:
a flexible sheet and a controller configured to control the flexible sheet, the flexible sheet comprises:
a first flexible layer;
a second flexible layer overlapped with the first flexible layer;
a plurality of functional layers sandwiched between the first flexible layer and the second flexible layer, wherein each of the plurality of functional layer comprises a carbon nanotube layer, the carbon nanotube layer comprises a plurality of carbon nanotubes uniformly distributed in the carbon nanotube layer; and
a plurality of electrodes, wherein two ends of each of the plurality of electrodes are separately electrically connected with a pair of the plurality of functional layers located a part from each other, and a voltage is applied on each two electrodes of the plurality of electrodes, a circuit is formed between the controller, the two of the plurality of electrodes , the plurality of functional layers electrically connected with the two of the plurality of electrodes , and skin of a user, a current flows through the controller , the two of the plurality of electrodes, the plurality of functional layers electrically connected with the two of the plurality of electrodes, and the skin of the user.

2. The soft physiotherapy instrument of claim 1, wherein the first flexible layer or the second flexible layer defines a window, and each of the at least one first electrode lead and the at least one second electrode lead is exposed from the window and electrically connected to the controller.

3. The soft physiotherapy instrument of claim 1, wherein the second flexible layer is directly attached on a user's face, the second flexible layer is a porous structure with a plurality of micropores.

4. The soft physiotherapy instrument of claim 3, wherein the plurality of functional layers are located at a forehead position, a cheek position, or a chin position.

5. The soft physiotherapy instrument of claim 1, wherein K is the numbering of each of the plurality of electrodes, a voltage is applied on each two electrodes in an order 1 and 2, 2 and 3, 3 and 4 ... K-1 and K.

6. The soft physiotherapy instrument of claim 5, wherein two pairs of the plurality of functional layers corresponding to each two electrodes are cyclically input current, and face skin corresponding the two pairs of the plurality of functional layers are cyclically stimulated.

7. The soft physiotherapy instrument of claim 1, wherein each of the plurality of functional layers further comprises a graphene layer overlapped with the carbon nanotube layer, the graphene layer comprises a plurality of graphenes.

8. The soft physiotherapy instrument of claim 1, wherein the carbon nanotube layer comprises a carbon nanotube film or a plurality of carbon nanotube films overlapped with each other, the carbon nanotube film is a freestanding film.

9. The soft physiotherapy instrument of claim 8, wherein the carbon nanotube film comprises a plurality of successive and oriented carbon nanotubes joined end-to-end by van der Waals attractive force therebetween.

10. The soft physiotherapy instrument of claim 8, wherein the carbon nanotube film comprises a plurality of successively oriented carbon nanotube segments joined end-to-end by van der Waals attractive force therebetween, and each carbon nanotube segment comprises a plurality of carbon nanotubes substantially parallel to each other, and joined by van der Waals attractive force therebetween.

## Patentansprüche

1. Weiches Physiotherapie-Instrument, umfassend:
ein flexibles Tuch und eine Steuerung, die zum Steuern des flexiblen Tuches konfiguriert ist, wobei das flexible Tuch Folgendes umfasst:
eine erste flexible Schicht;
eine zweite flexible Schicht, die mit der ersten flexiblen Schicht überlappt;
eine Vielzahl von Funktionsschichten, die zwischen der ersten flexiblen Schicht und der zweiten flexiblen Schicht angeordnet sind, wobei jede der Vielzahl von Funktionsschichten eine Kohlenstoffnanoröhrenschicht umfasst, wobei die Kohlenstoffnanoröhrenschicht eine Vielzahl von Kohlenstoffnanoröhren umfasst, die gleichmäßig in der Kohlenstoffnanoröhrenschicht verteilt sind; und
eine Vielzahl von Elektroden, wobei die beiden Enden jeder der Vielzahl von Elektroden separat elektrisch mit einem Paar der Vielzahl von Funktionsschichten verbunden sind, die voneinander entfernt liegen, und eine Spannung an jeweils zwei Elektroden der Vielzahl von Elektroden angelegt wird, wobei ein Stromkreis zwischen der Steuerung, den beiden der Vielzahl von Elektroden, den Vielzahl von Funktionsschichten, die elektrisch mit den beiden der Vielzahl von Elektroden verbunden sind, und der Haut des Benutzers gebildet wird, wobei Strom durch die Steuerung, die beiden der Vielzahl von Elektroden, die Vielzahl von Funktionsschichten, die elektrisch mit den beiden der Vielzahl von Elektroden verbunden sind, und die Haut des Benutzers fließt.

2. Weiches Physiotherapie-Instrument nach Anspruch 1, wobei die erste flexible Schicht oder die zweite flexible Schicht ein Fenster aufweist und jede der mindestens einen ersten Elektrodenleitung und der mindestens einen zweiten Elektrodenleitung am Fenster freiliegt und elektrisch mit der Steuerung verbunden ist.

3. Weiches Physiotherapie-Instrument nach Anspruch 1, wobei die zweite flexible Schicht direkt auf dem Gesicht des Benutzers angebracht ist, und die zweite flexible Schicht eine poröse Struktur mit einer Vielzahl von Mikroporen ist.

4. Weiches Physiotherapie-Instrument nach Anspruch 3, wobei sich die Vielzahl von Funktionsschichten an Stirn-, Wangen- oder Kinnposition befinden.

5. Weiches Physiotherapie-Instrument nach Anspruch 1, wobei K die Nummerierung jeder der Vielzahl von Elektroden ist, und eine Spannung an jeweils zwei Elektroden in der Reihenfolge 1 und 2, 2 und 3, 3 und 4 ... K -1 und K angelegt wird.

6. Weiches Physiotherapie-Instrument nach Anspruch 5, wobei den zwei Paaren der Vielzahl von Funktionsschichten, die jeweils zwei Elektroden entsprechen, zyklisch Strom zugeführt wird, und die Gesichtshaut, die den beiden Paaren der Vielzahl von Funktionsschichten entspricht, zyklisch stimuliert wird.

7. Weiches Physiotherapie-Instrument nach Anspruch 1, wobei jede der Vielzahl von Funktionsschichten außerdem eine Graphenschicht umfasst, die mit der Kohlenstoffnanoröhrenschicht überlappt ist, wobei die Graphenschicht eine Vielzahl von Graphene umfasst.

8. Weiches Physiotherapie-Instrument nach Anspruch 1, wobei die Kohlenstoffnanoröhrenschicht einen Kohlenstoffnanoröhrenfilm oder eine Vielzahl von Kohlenstoffnanoröhrenfilmen, die sich überlappen, umfasst, wobei der Kohlenstoffnanoröhrenfilm ein freistehender Film ist.

9. Weiches Physiotherapie-Instrument nach Anspruch 8, wobei der Kohlenstoffnanoröhrenfilm eine Vielzahl von aufeinanderfolgenden Kohlenstoffnanoröhren umfasst, die von Ende zu Ende durch Van-der-Waals-Anziehungskraft miteinander verbunden sind.

10. Weiches Physiotherapie-Instrument nach Anspruch 8, wobei der Kohlenstoffnanoröhrenfilm eine Vielzahl von aufeinanderfolgenden Kohlenstoffnanoröhrchensegmenten umfasst, die von Ende zu Ende durch Van-der-Waals-Anziehungskraft miteinander verbunden sind, und jedes Kohlenstoffnanoröhrchensegment eine Vielzahl von Kohlenstoffnanoröhren umfasst, die im Wesentlichen parallel zueinander verlaufen und durch die Van-der-Waals-Anziehungskraft miteinander verbunden sind.

## Revendications

1. Instrument de physiothérapie souple, comprenant :
une feuille souple et un contrôleur configuré pour commander la feuille souple, la feuille souple comprend :
une première couche souple ;
une seconde couche flexible superposée à la première couche flexible ;
une pluralité de couches fonctionnelles prises en sandwich entre la première couche flexible et la seconde couche flexible, dans laquelle chacune de la pluralité de couches fonctionnelles comprend une couche de nanotubes de carbone, la couche de nanotubes de carbone comprenant une pluralité de nanotubes de carbone uniformément répartis dans la couche de nanotubes de carbone ; et
une pluralité d'électrodes, dans laquelle les deux extrémités de chacune des électrodes sont connectées électriquement à une paire de couches fonctionnelles situées à une certaine distance l'une de l'autre, et une tension est appliquée sur chacune des deux électrodes de la pluralité d'électrodes, un circuit est formé entre le contrôleur et les deux électrodes de la pluralité d'électrodes, la pluralité de couches fonctionnelles connectées électriquement aux deux électrodes de la pluralité, et la peau d'un utilisateur, un courant circule à travers le contrôleur, les deux électrodes de la pluralité, la pluralité de couches fonctionnelles connectées électriquement aux deux électrodes de la pluralité, et la peau de l'utilisateur.

2. Instrument de physiothérapie souple selon la revendication 1, dans lequel la première couche flexible ou la seconde couche flexible définit une fenêtre, et chacun des au moins un fil de la première électrode et au moins un fil de la seconde électrode est exposé à partir de la fenêtre et connecté électriquement au contrôleur.

3. Instrument de physiothérapie souple de la revendication 1, dans lequel la seconde couche flexible est directement fixée sur le visage de l'utilisateur, la seconde couche flexible est une structure poreuse avec une pluralité de micropores.

4. Instrument de physiothérapie souple de la revendication 3, dans lequel la pluralité de couches fonctionnelles est située au niveau du front, des joues ou du menton.

5. L'instrument de physiothérapie douce de la revendication 1, dans lequel K est la numérotation de chacune de la pluralité d'électrodes, une tension est appliquée sur chacune des deux électrodes dans l'ordre 1 et 2, 2 et 3, 3 et 4 ... K- 1 et K.

6. L'instrument de physiothérapie douce de la revendication 5, dans lequel deux paires de la pluralité de couches fonctionnelles
correspondant à chacune des deux électrodes sont alimentées cycliquement en courant, et la peau du visage correspondant aux deux paires de la pluralité de couches fonctionnelles est stimulée cycliquement.

7. L'instrument de physiothérapie souple de la revendication 1, dans lequel chacune des couches fonctionnelles comprend en outre une couche de graphène superposée à la couche de nanotubes de carbone, la couche de graphène comprenant une pluralité de graphènes.

8. Instrument de physiothérapie souple selon la revendication 1, dans lequel la couche de nanotubes de carbone comprend un film de nanotubes de carbone ou une pluralité de films de nanotubes de carbone superposés les uns aux autres, le film de nanotubes de carbone est un film autoportant.

9. Instrument de physiothérapie souple selon la revendication 8, dans lequel le film de nanotubes de carbone comprend une pluralité de nanotubes de carbone successifs et orientés joints bout à bout par la force d'attraction de van der Waals entre eux.

10. Instrument de physiothérapie souple selon la revendication 8, dans lequel le film de nanotubes de carbone comprend une pluralité de segments de nanotubes de carbone orientés successivement joints bout à bout par la force d'attraction de van der Waals entre eux, et chaque segment de nanotubes de carbone comprend une pluralité de nanotubes de carbone sensiblement parallèles les uns aux autres, et joints par la force d'attraction de van der Waals entre eux.
